# EUROPEAN PATENT SPECIFICATION

(11) **EP 0 823 260 B1**
(45) Date of publication and mention of the grant of the patent: **29.05.2002**
(21) Application number: 97305985.0
(22) Date of filing: 06.08.1997
(51) Int. Cl.: A61M 16/00

(54) **Mouth-to-mouth resuscitation device**
Mund-zu-Mund Wiederbelebungsgerät
Appareil de réanimation bouche à bouche

(30) Priority: 06.08.1996 ZA 9606670
(43) Date of publication of application: 11.02.1998
(73) Proprietor: Jones, Paul Leonard, Bluff 4052 (ZA); Wolmarans, Josephus Theodorus Johannes, Moseley Park, Pinetown 3610 (ZA); Jones, Leonard Frederic Eric, Bluff 4052 (ZA)
(72) Inventor: Jones, Paul Leonard, Bluff, 4052 (ZA)
(74) Representative: Walsh, David Patrick

(56) References cited:
- EP-A- 0 484 684
- EP-A- 0 710 488
- US-A- 5 005 568
- US-A- 5 295 478

## Description

The present invention relates to a device for use in mouth-to-mouth resuscitation and, in particular, to a device which allows effective mouth-to-mouth resuscitation to be given while protecting the resuscitator from fluid contact with the patient.

Mouth-to-mouth resuscitation is a commonly used expired-air resuscitation technique. There is a reluctance on the part of emergency services personnel, for example, ambulance crews and police personnel, to perform mouth to mouth resuscitation unprotected, due primarily to the spread of Acquired Immune Deficiency Syndrome. A number of devices for this purpose are available, however, these devices have been found to be unsatisfactory in providing protection for a resuscitator performing mouth-to-mouth resuscitation on a patient.

Conventional mouth-to-mouth resuscitation devices comprise two mouthpieces; one to be received in the patient's mouth, the other in the resuscitator's mouth, and a plastics sheet extending between the mouthpieces and from the periphery of the device. The patient's mouthpiece has a large through aperture and the resuscitator's mouthpiece has two smaller spaced apart through apertures. The plastics sheet extends across the two resuscitator's apertures but has a hole therein located between the two apertures so that air flow through the two apertures passes along the resuscitator's side of the sheet, through the hole and through the aperture in the patient's mouthpiece. The patient's exhalation on the other hand is prevented from passing through the hole in the sheet since the hole abuts the adjacent region of the resuscitator's mouthpiece.

Another known device is illustrated in GB 2204498 and comprises two mouthpieces having a value member mounted therebetween. The valve member comprises a disc which is seated on one mouthpiece when in the resuscitation position. Channels are provided in that mouthpiece to allow air flow between the periphery of the disc and the mouthpiece since the disc itself lies directly in the path of the incoming air. Exhalation by the patient causes the disc to close off the opening in the other mouthpiece to prevent the passage of air through the device. Thus, the disc can freely oscillate between the open and closed positions as the resuscitator and the patient exhale.

EP 0 710 488 A1 describes a similar device exhibiting the same draw-back, i.e. air has to flow around the outer edges of the valve and so the resuscitator has to blow harder to achieve an adequate pressure between the mouthpieces.

A disadvantage with these known devices is that the plastics sheet or valve member lies directly in the path of the incoming air so that optimum efficiency cannot be achieved. Furthermore, the sheet aperture or through channels allow unprotected air flow through the device so that little or no protection is afforded to the patient against fluid contact with the resuscitator. This can be a problem since the resuscitator's exhalation may include undesirable fluids such as moisture, saliva or the like.

Furthermore, it has been found that certain known devices for use in mouth-to-mouth resuscitation, do not completely prevent the passage of bodily fluids entrained in exhaled air from a patient from reaching a resuscitator.

The invention has been made from a consideration of the disadvantages with known devices and in order to provide an improved mouth-to-mouth resuscitation device. One preferred object of the invention is to provide a device for use in mouth-to-mouth resuscitation having a valve member which does not substantially restrict the air flow through the device when in the resuscitation position. A further preferred object of the invention is to provide a mouth-to-mouth resuscitation device which provides some protection for the patient against contact with undesirable fluid emission from the resuscitator.

A further preferred object of the invention is to provide a mouth-to-mouth resuscitation device which effectively prevents the passage of any bodily fluids from reaching the resuscitator.

According to a first aspect of the invention there is provided a device for use in mouth-to-mouth resuscitation comprising first means adapted to be received in or covered by a patient's mouth, second means adapted to be received in or covered by the resuscitator's mout, the first and second means defining a passage through the device, and valve means located therebetween wherein the valve means is pivotally mounted relative to one of said first and second means and is moveable between a first position in which fluid flow through the device is facilitated and a second position in which fluid flow through the device is restricted, characterised in that the valve means extends from its pivotal mounting substantially in the direction of the passage when in the first position.

Preferably, the valve means is pivotable between said first and second positions. Preferably the valve means comprises a disc, which may for example be circular and substantially planar, and a longitudinal element such as a pin or pins secured to or integrally formed with the disc. Preferably, the disc is pivotable about an axis of said longitudinal element. Preferably, the disc is impermeable to fluid, particularly air. Preferably, the longitudinal element is located at one side of the disc or on the peripheral edge of the disc. Preferably, the valve means or disc comprises a fluid impermeable material such as plastics.

Preferably the first and second means form part of first and second bodies which are releasably secured together by an interlocking arrangement. Preferably the valve means is mounted on one of the first or second bodies for example by location of the longitudinal element in one or more channels formed in such body. Typically a through aperture is provided between the first and second means and the valve means acts to close off the aperture when in the first position, for example by the disc extending across the aperture. Preferably, the first and second means and the valve means are of rigid synthetic plastics material.

A protective sheet or barrier such as of plastics material may be provided on the device, to protect the resuscitator and patient from fluid contact around the exterior of the device. Thus, for example, the sheet may comprise a suitably dimensioned sheet having an aperture formed therein through which the device is received, the sheet being held between the first and second bodies. The sheet then extends radially outwardly from the body of the device and physically isolates the patient from the resuscitator. The sheet preferably comprises a flexible or semi-flexible material. Preferably, the sheet or barrier is impermeable to fluid.

The sheet or barrier of the resuscitation device may be in the form of a substantially circular sheet of flexible synthetic plastics material that is deformable to conform to the facial contour of a patient. Preferably, the device comprises filter means which, in use, acts to filter undesirable fluids from the resuscitator's exhalation. Preferably, the filter means is located in or on an aperture or extends across the passage defined by said first and second means. The filter means may be as described below in connection with the second aspect of the invention. Preferably, the filter means comprises a liquid impervious element which is pervious to air thus allowing the free passage of air therethrough. Preferably, the filter means is in the form of a sheet.

Preferably the first and second means comprise a through aperture and the filter means is located in or on said aperture and spans said aperture at that location.

Preferably the filter means is mounted between said first and second means. The filter means may be mounted in the second means and held in place by suitable retaining means such as a ring or bush. The first means may then be secured to the second means. Preferably the filter means comprises a mesh. The structure of the mesh and the dimensions of the mesh apertures may be selected to give the desired degree of filtering while maintaining adequate throughflow of oxygen from the resuscitator to the patient. The mesh may be solid or semi-flexible but is preferably flexible and is preferably of sufficient size to adequately span the aperture or internal cavity of the first or second means. The filter means may be as described above.

The valve means may be in the form of an impermeable disc that is hingedly mounted within the passage to be displaceable between a first position wherein the disc allows free passage of air from the second to the first mouthpiece section, and a second position wherein the disc prevents the passage of air from the first to second mouthpiece section; and a flexible, impermeable barrier that is secured to at least one of the mouthpiece sections and that extends radially outwardly therefrom.

In use, the resuscitator places the first means in the patient's mouth and ensures the protective sheet or barrier extends over the patient's facial region. The resuscitator pinches closed the patient's nose through the protective sheet and covers the second means with his mouth. Upon blowing through the device, the resuscitator's breath is filtered by the filter means, passes through the second means and causes the valve member to move to the first position so that free passage of air to the patient is facilitated. Upon subsequent compression of the patient's lungs, the patient's breath causes the valve member to move to the second position so that the patient's breath is prevented from passing through the device to the resuscitator and instead escapes on the patient's side of the protective sheet.

Accordingly, body fluids that may be entrained in the exhaled air of the patient are thus prevented from reaching the resuscitator. It has been found further that the barrier or protection sheet also provides the resuscitator with protection against facial bleeding of a patient and against mucus and vomit from the patient. Furthermore, the filter means or liquid impervious element prevents the passage of any bodily fluids from reaching the resuscitator.

Typically the device of the invention is used once and then disposed of.

The invention will now be described further by way of example only and with reference to the accompanying drawings in which:

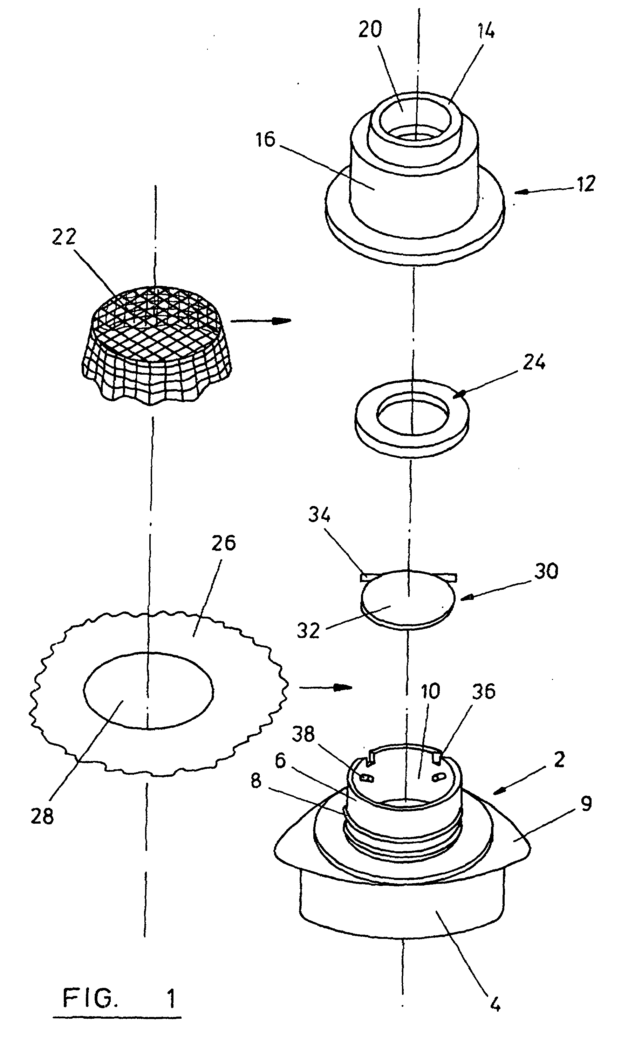

Fig.1 is an exploded view of a device of the invention;

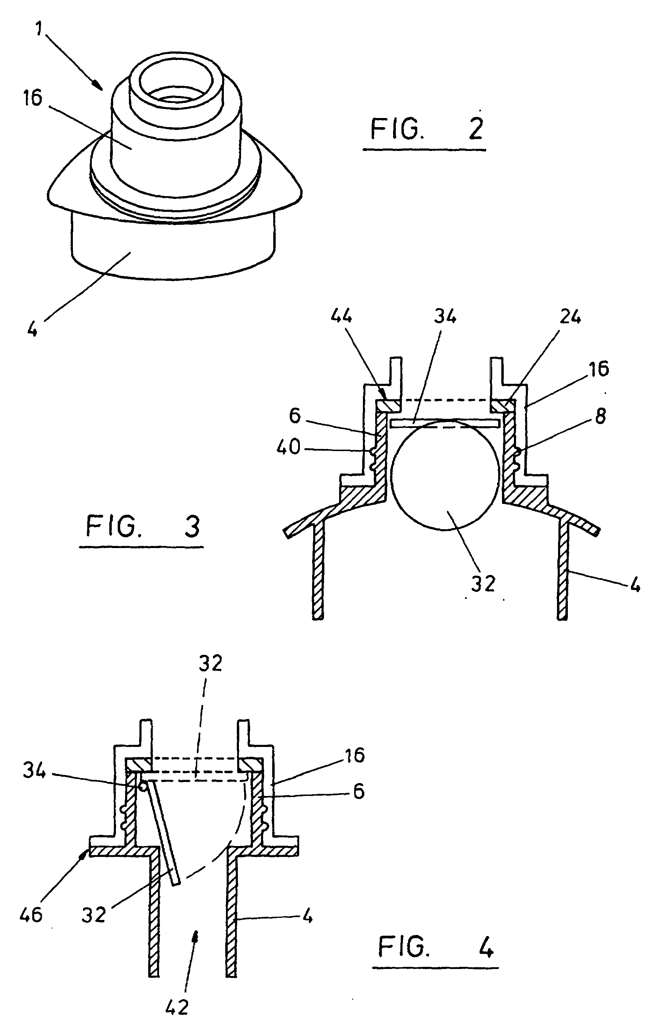

Fig. 2 is a view of the assembled device corresponding to Fig. 1;
Fig. 3 is a front sectional view showing the valve member in the open position;
Fig. 4 is a side sectional view showing the valve member in the open and closed positions;

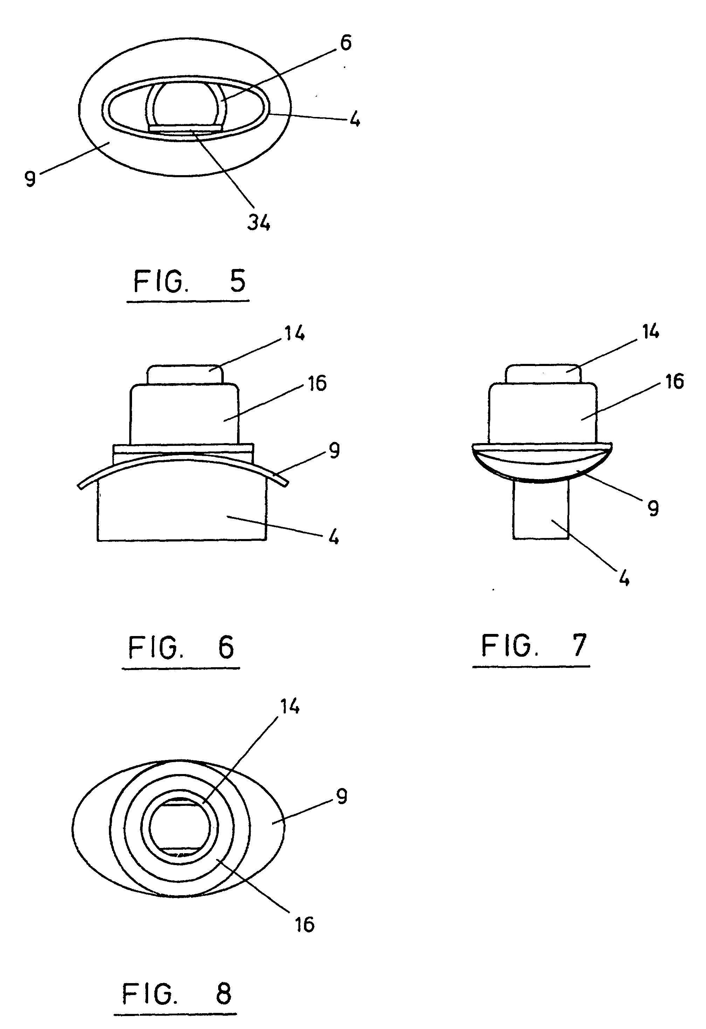

Fig. 5 is an underplan view of the device;
Fig. 6 is a front view of the device;
Fig. 7 is a side view of the device; and
Fig. 8 is a plan view of the device;

Referring to the drawings, a mouth-to-mouth resuscitation device 1 comprises a first body or first mouthpiece section 2, having a mouthpiece 4 to be received in a patient's mouth and a body portion 6 extending therefrom. Ribs 8 are provided on the body portion 6 for engagement with corresponding grooves 40 provided on the interior of body portion 16 of a second body or second mouthpiece section 12. The second body 12 has a mouthpiece 14 to be received in a resuscitator's mouth. Each of the first and second bodies 2, 12 have through apertures 10, 20 respectively extending therethrough so that when assembled the device has a continuous through aperture 42 extending down the centre thereof. The first body 2 also includes a protective flange 9.

The mouthpiece sections 2 and 12 are of moulded synthetic plastics material. The free end of the first mouthpiece section 2 is of oval configuration, with the first mouthpiece section 2 defining an oval mouthpiece 4 that extends from a curved oval flange 9. A cylindrical boss 6 extends from a side of the flange 9 opposite to the side from which the mouthpiece 4 extends. The boss 6 defines an internal passageway section and has a first engagement formation in the form of a pair of annular ribs 8 formed on the outer circumference thereof. The boss 6 defines a pair of stop formations in the form of projections 38 that extend operatively inwardly from an internal wall of the boss 6. The purpose of the projections 38 will become apparent hereinafter. Furthermore, the boss 6 defines a pair of hinge support formations 36 for hingedly supporting the disc 32 in a manner that will become apparent hereinafter.

The second mouthpiece section 12 defines a cylindrical mouthpiece 16 and defines an internal passageway section. As such, the second mouthpiece section 12 has an internal diameter that is slightly larger than the external diameter of the boss 6 of the first mouthpiece section 2 and defines a second engagement formation in the form of a pair of annular grooves 40 defined in an internal wall thereof that define complementary configurations to that of the ribs 8 of the boss 6. Accordingly, the boss 6 of the first mouthpiece section is inserted into the second mouthpiece section 12 in spigot and socket-fashion in an arrangement wherein the ribs 8 fit into and engage with the grooves 40 of the first mouthpiece section. As such, in their engaged configuration, the first and second mouthpiece sections define an internal passage 42.

The second mouthpiece section 12 defines a step formation that forms an internal shoulder formation 44. The purpose of the shoulder formation 44 is explained hereinafter.

The resuscitation device 1 includes an annular element 24 that is located between the first mouthpiece section 2 and the second mouthpiece section 12 when they are engaged with one another. More particularly, the annular element 24 is located in abutment with the shoulder formation 44 of the second mouthpiece section 12 as shown in Figures 3 and 4 of the drawings. The disc 32 includes a hinge member 34 that is hingedly mounted between the operative under side of the annular element 24 and the hinge support formations 36 of the boss 6, with the annular element 24 providing a valve seating on its operative under side. As such, the disc 32 is hingedly displaceable between a first position in which free air flow through the passage 42 is permitted, and a second position in which the disc 32 blocks off the passage and prevents air flow from the first mouthpiece section 2 to the second mouthpiece section 12. More particularly, when the disc 32 is in its second position, it is in sealing engagement with the valve seating of the annular element 24 thereby preventing the passage of air from the first to the second mouthpiece section.

A filter 22 is located within the interior of the body 12 and spans the aperture 20. The filter 22 is held in place by the annular member 24. Typically the filter 22 is located adjacent the mouthpiece 14. The filter or liquid impervious element 22 is of a material that allows the free passage of air therethrough. Preferably, a non-woven material is used. The element 22 is in the form of a sheet element and extends across the passage 42 defined by the first and second mouthpiece sections. As such, the element 22 is of flexible deformable material and is secured between the annular element 24 and the step formation 44 of the second mouthpiece section 12. A protective sheet 26 has an aperture 28 through which the body portion 6 is received before being engaged with the body portion 16. Thus, the protective sheet extends around the outside of the device to provide protection against fluid contact. The protective sheet 26 does not interfere with the aperture 10, 20. Typically the protective sheet comprises a flexible impermeable barrier. The barrier 26 has an inner circular opening 28 that is a close fit around the boss 6. More particularly, the first and second mouthpiece sections define an interface region 46 between them with the barrier 26 being secured between the first and second mouthpiece sections at the. interface region 46 to extend radially outwardly therefrom.

A valve member 30 comprises a disc 32 having a pin 34 located on a peripheral edge thereof. The pin 34 is mounted on the body portion 6 by locating each end of the pin in a corresponding open channel 36 provided on the upper edge of the body portion 6. The disc 32 is pivotable about the axis of the pin 34 between an open position in which the disc lies at least partially in the direction of the aperture 10 and a closed position in which the disc spans the aperture 10 thereby closing off the aperture. Such action is more clearly illustrated in Figs. 3 and 4. Typically the disc is impermeable.

The device is assembled by locating the barrier 26 on the boss 6 of the first mouthpiece section 2. Thereafter, the disc 32 is located, via its hinge member 34, on the hinge support formations 36 of the boss 6. The annular element 24 is located on the boss 6 of the first mouthpiece section 2, thereby forming a hinge about which the disc 32 is hingedly displaceable. The liquid-impervious sheet element 22 is located on the annular element 24 to extend across it, and the second mouthpiece section 12 is then fitted onto the boss 6 of the first mouthpiece section 2 and engaged therewith, effectively securing the annular element 24, the sheet element 22 and the barrier 26 between them.

In use, the first mouthpiece section - 2 of the resuscitation device 1 is fitted into a patient's mouth and the lower facial region of the patient, including the nose, is covered by the carrier 26. The patient's nose is pinched through the barrier 26 and the resuscitator can then proceed with mouth-to-mouth resuscitation by placing his mouth over the second mouthpiece 12 and blowing into the device into the passage 42. The disc 32 being hingedly mounted on the under side of the annular element 24, permits the free flow of air into the patient by being displaced operatively downwardly due to air pressure acting on the disc 32 as is apparent from Figure 4 of the drawings. The projections 38 prevent the disc 32 from being displaced downwardly through a full 90° angle, in order to ensure thereby that the disc 32 presents sufficient surface area for exhaled air from a patient to act on the disc 32 to displace it in its second position. During exhalation by the patient, the pressure of the exhaled air forces the disc 32 into its second position in which it is in sealing engagement with the operative under side of the annular element 24, thereby preventing the flow of air into the second mouthpiece section 12. Accordingly, exhaled air is diverted around the first mouthpiece section onto the patient's side of the barrier. Body fluids that may be entrained in the exhaled air of the patient are thus prevented from reaching the resuscitator. Furthermore, it has been found that the barrier 26 also provides the resuscitator with protection against facial bleeding of a patient and against mucus and vomit from the patient.

An important preferred advantage of the resuscitation device 1 is that the liquid impervious element 22 prevents bodily fluids from a patient being transferred via the passage 42 defined by the first and second mouthpiece sections and vice-versa.

The resuscitation device of the invention provides effective protection for a resuscitator against diseases, such as, Acquired Immune Deficiency Syndrome that may be transmitted via a patient's bodily fluids to a resuscitator while performing mouth-to-mouth resuscitation on the patient.

It will be appreciated that the present invention is not intended to be restricted to the details of the above embodiment which is described by way of example only.

## Claims

1. A device (1) for use in mouth-to-mouth resuscitation comprising first means (4) adapted to be received in or covered by a patient's mouth, second means (14) adapted to be received in or covered by the resuscitator's mouth, the first and second means defining a passage (42) through the device, and valve means (30) located therebetween wherein the valve means (30) is pivotally mounted relative to one of said first (4) and second (14) means and is moveable between a first position in which fluid flow through the device (1) is facilitated and a second position in which fluid flow through the device is restricted, **characterised in that** the valve means extends from its pivotal mounting substantially in the direction of the passage (42) when in the first position.

2. A device (1) for use in mouth-to-mouth resuscitation according to claim 1, wherein the valve means (30) comprises a disc (32).

3. A device (1) for use in mouth-to-mouth resuscitation according to any preceding claim, wherein the valve means (30) comprises a longitudinal element (34) defining the pivot axis.

4. A device (1) for use in mouth-to-mouth resuscitation according to claim 3, wherein the longitudinal element (34) is located at one side of the disc.

5. A device (1) for use in mouth-to-mouth resuscitation according to any preceding claim, wherein the valve (30) means acts to close off the passage (42) when in the second position.

6. A device (1) for use in mouth-to-mouth resuscitation according to any preceding claim, wherein the device (1) comprises filter means (22) which, in use, acts to filter undesirable fluids from the resuscitator's exhalation.

7. A device (1) for use in mouth-to-mouth resuscitation according to claim 6, wherein the filter means (22) comprises a liquid impervious element which is pervious to air.

8. A device (1) for use in mouth-to-mouth resuscitation according to claim 6 or claim 7, wherein the filter means (22) is located in or on said passage (42) and spans said passage at that location.

9. A device (1) for use in mouth-to-mouth resuscitation according to claim 6, 7 or 8, wherein the filter means (22) comprises a mesh.

10. A device (1) for use in mouth-to-mouth resuscitation according to any preceding claim wherein the valve means (30) is in the form of an impermeable disc (32) that is hingedly mounted within the passage (42) to be displaceable between a first position wherein the disc (32) allows free passage of air from the second to the first mouthpiece section, and a second position wherein the disc prevents the passage of air from the first to second mouthpiece section; and a flexible, impermeable barrier that is secured to at least one of the mouthpiece sections and that extends radially outwardly therefrom.

## Patentansprüche

1. Vorrichtung (1) zur Mund-zu-Mund-Wiederbelebung, welche ein zum Einsetzen in den Mund des Patienten oder zum Abdecken seines Mundes ausgebildetes erstes Mittel (4), ein zweites zum Einsetzen in den Mund des Wiederbelebers oder zum Abdecken seines Mundes ausgebildetes Mittel (14), wobei das erste und zweite Mittel einen Durchlass (42) durch die Vorrichtung festlegen, und ein dazwischen angeordnetes Ventilmittel (30) aufweist, wobei das Ventilmittel (30) schwenkbar in Bezug auf eines der genannten ersten (4) und zweiten (14) Mittel befestigt ist und zwischen einer ersten Stellung, in der ein Durchfluss durch die Vorrichtung möglich ist, und einer zweiten Stellung, in der der Durchfluss durch die Vorrichtung beschränkt ist, beweglich ist, **dadurch gekennzeichnet, dass** das Ventilmittel sich von seiner schwenkbaren Befestigung im Wesentlichen in Richtung des Durchlasses (42) erstreckt, wenn es sich in der ersten Stellung befindet.

2. Vorrichtung (1) zur Mund-zu-Mund-Wiederbelebung nach Anspruch 1, bei der das Ventilmittel (30) eine Scheibe (32) aufweist.

3. Vorrichtung (1) zur Mund-zu-Mund-Wiederbelebung nach einem der vorangehenden Ansprüche, bei der das Ventilmittel (30) ein längliches Bauteil (34) aufweist, welches die Schwenkachse bildet.

4. Vorrichtung (1) zur Mund-zu-Mund-Wiederbelebung nach Anspruch 3, bei der das längliche Bauteil (34) an einer Seite der Scheibe angeordnet ist.

5. Vorrichtung (1) zur Mund-zu-Mund-Wiederbelebung nach einem der vorangehenden Ansprüche, bei dem das Ventilmittel (30) dazu dient, den Durchlass (42) zu verschließen, wenn es sich in der zweiten Stellung befindet.

6. Vorrichtung (1) zur Mund-zu-Mund-Wiederbelebung nach einem der vorangehenden Ansprüche, bei der die Vorrichtung (1) ein Filtermittel (22) aufweist, welches bei Gebrauch dazu dient, unerwünschte Flüssigkeiten aus dem Atmen des Wiederbelebers zu filtern.

7. Vorrichtung (1) zur Mund-zu-Mund-Wiederbelebung nach Anspruch 6, bei der das Filtermittel (22) ein für Flüssigkeit undurchlässiges Bauteil aufweist, welches für Luft durchlässig ist.

8. Vorrichtung (1) zur Mund-zu-Mund-Wiederbelebung nach Anspruch 6 oder 7, bei der das Filtermittel (22) in oder auf dem genannten Durchlass (42) angeordnet ist und an dieser Stelle den genannten Durchlass überbrückt.

9. Vorrichtung (1) zur Mund-zu-Mund-Wiederbelebung nach den Ansprüchen 6, 7 oder 8, bei der das Filtermittel (22) ein Sieb aufweist.

10. Vorrichtung (1) zur Mund-zu-Mund-Wiederbelebung nach einem der vorangehenden Ansprüche, bei der das Ventilmittel (30) die Form einer undurchlässigen Scheibe (32) hat, die scharnierartig in dem Durchlass (42) befestigt ist, um zwischen einer ersten Stellung, in der die Scheibe (32) einen freien Durchlass von Luft von dem zweiten zu dem ersten Mundstückbereich ermöglicht, und einer zweiten Stellung, in der die Scheibe den Durchlass von Luft von dem ersten zu dem zweiten Mundstückbereich verhindert, beweglich zu sein, und bei der die Vorrichtung eine flexible, undurchlässige Absperrung hat, welche an zumindest einer der Mundstückbereiche befestigt ist und die sich radial von diesem weg erstreckt.

## Revendications

1. Un dispositif (1) destiné à être utilisé dans la réanimation bouche à bouche comprenant un premier moyen (4) agencé pour être reçu dans la bouche d'un patient ou recouvert par celle-ci, un deuxième moyen (14) agencé pour être reçu dans la bouche du réanimateur ou recouvert par celle-ci, les premier et deuxième moyens définissant un passage (42) au travers du dispositif et un moyen à clapet (30) situé entre eux, dans lequel le moyen à clapet (30) est monté de façon pivotante par rapport à l'un dudit premier moyen (4) et dudit deuxième moyen (14) et est apte à être déplacé entre une première position dans laquelle l'écoulement de fluide au travers du dispositif (1) est facilité et une deuxième position dans laquelle l'écoulement de fluide au travers du dispositif est limité, **caractérisé en ce que** le moyen à clapet s'étend depuis son montage pivotant essentiellement dans la direction du passage (42) lorsqu'il est dans la première position.

2. Un dispositif (1) destiné à être utilisé dans la réanimation bouche à bouche selon la revendication 1, dans lequel le moyen à clapet (30) comprend un disque (32).

3. Un dispositif (1) destiné à être utilisé dans la réanimation bouche à bouche selon l'une quelconque des revendications précédentes, dans lequel le moyen à clapet (30) comprend un élément longitudinal (34) définissant l'axe de pivotement.

4. Un dispositif (1) destiné à être utilisé dans la réanimation bouche à bouche selon la revendication 3, dans lequel l'élément longitudinal (34) est situé sur un côté du disque.

5. Un dispositif (1) destiné à être utilisé dans la réanimation bouche à bouche selon l'une quelconque des revendications précédentes, dans lequel le moyen à clapet (30) agit pour fermer le passage (42) lorsqu'il est dans la seconde position.

6. Un dispositif (1) destiné à être utilisé dans la réanimation bouche à bouche selon l'une quelconque des revendications précédentes, dans lequel le dispositif (1) comprend un moyen à filtre (22) qui, en service, agit pour filtrer les fluides indésirables à partir de l'exhalation du réanimateur.

7. Un dispositif (1) destiné à être utilisé dans la réanimation bouche à bouche selon la revendication 6, dans lequel le moyen à filtre (22) comprend un élément imperméable aux liquides qui est perméable à l'air.

8. Un dispositif (1) destiné à être utilisé dans la réanimation bouche à bouche selon la revendication 6 ou la revendication 7, dans lequel le moyen à filtre (22) est situé dans ou sur ledit passage (42) et chevauche ledit passage en cet emplacement.

9. Un dispositif (1) destiné à être utilisé dans la réanimation bouche à bouche selon la revendication 6, 7 ou 8, dans lequel le moyen à filtre (22) comprend un treillis.

10. Un dispositif (1) destiné à être utilisé dans la réanimation bouche à bouche selon l'une quelconque des revendications précédentes, dans lequel le moyen à clapet (30) est sous la forme d'un disque imperméable (32) qui est monté de façon pivotante dans le passage (42) pour être apte à être déplacé entre une première position dans laquelle le disque (32) permet un passage libre de l'air depuis la deuxième vers la première section de la pièce de bouche, et une deuxième position dans laquelle le disque empêche le passage de l'air de la première à la deuxième section de la pièce de bouche, et une barrière imperméable, souple qui est fixée à au moins l'une des sections de pièce de bouche et qui s'étend radialement vers l'extérieur à partir de celle-ci.
